**Europäisches Patentamt**

**European Patent Office**

**Office européen des brevets**

(11) Publication number: **0 241 573**
**A1**

## (12) EUROPEAN PATENT APPLICATION

(21) Application number: **86105293.4**

(22) Date of filing: **16.04.86**

(51) Int. Cl.4: **A61K 7/42** , **A61K 7/48**

(43) Date of publication of application:
**21.10.87 Bulletin 87/43**

(84) Designated Contracting States:
**AT BE CH DE FR GB IT LI LU NL SE**

(71) Applicant: **Sansho Seiyaku Co., Ltd.**
**26-7, Oike 2-chome**
**Ohnojo-shi Fukuoka-ken(JP)**

(72) Inventor: **Ohyama, Yasuaki**
**15-16, Oike 2-chome**
**Ohnojo-shi Fukuoka-ken(JP)**

(74) Representative: **Patentanwälte Müller-Boré,**
**Deufel, Schön, Hertel, Lewald, Otto**
**Postfach 26 02 47 Isartorplatz 6**
**D-8000 München 26(DE)**

(54) Topical agents for inhibiting the melanin generation.

(57) The topical agents incorporate vitamin E or a vitamin E derivative in an aqueous solution or suspension state. When the topical agents are applied to the skin lesion, the vitamin E or vitamin E derivative penetrates through the epidermis and is easily absorbed by melanocytes in the epidermic basal strutum thereby to remarkably inhibit the melanin generation by interfering the transfer of tyrosinase to premelanosome within the melanocytes.

EP 0 241 573 A1

## TOPICAL AGENTS FOR INHIBITING THE MELANIN GENERATION

Background of the Invention

The present invention relates to topical agents for inhibiting the melanin generation without inhibiting any tyrosinase activity, which have enabled easy skin absorption and cellular access to the target site, and which are used in the topical therapy for pigmentation such as chloasma.

Prior Art

As the therapy for pigmentation including chloasma, there has been carried out a method of continuous oral administration of vitamin C, etc., but when the case of highly pigmented, this often cannot be improved merely by the continuous oral administration of vitamin C. Further, since the pigmentation is regarded as an increase in amount of melanosome in the epidermis caused by the hyperfunction of melanocytes, there is a method for inhibiting excess generation of the melanin, where preventing influences of ultraviolet light which accelerates the melanocyte functions, that is, topical agents containing sunscreening agents are topically applied. As the sunscreens for the above described topical agents, there have been employed an ointment containing a substance which scatters light, such as powders of talc, zinc white, titanium oxide etc., an ointment containing an ultraviolet absorber such as paraaminobenzoic acid etc., and the like. These sunscreens have an object to prevent the hyperfunction of the melanocytes caused by the ultraviolet light. Further, it has also been known to use as a topical agent for the therapy for the pigmentation, a topical agent containing a substance having an effect to inhibit the melanin generation in the epidermis such as hydroquinone, hydroquinone derivatives, kojic acid etc.

On the other hand, vitamin E is mainly employed as an oral agent. Further, it is a medicine for ameliorating various disorders due to the impediment in peripheral blood circulation and is commercially available as a medicine for "frostbite" as an ointment by dissolving vitamin E in an oil phase.

In the above described prior art, sunscreening agents used in the topical agents in the topical therapy for the pigmentation are used in order to prevent the increase in the pigmentation by sunlight, and thus are to be used in combination with other therapy, and the effect as a topical agent for the pigmentation cannot be expected by the single used of such agents.

Topical agents used as declorizing agents such as hydroquinone, hydroquinone derivatives, e.g., hydroquinone monobenzyl ether etc., have a possibility to cause spotty or reticuler leucoderma, and therefore are not preferred as topical agents for the pigmentation. Topical agents containing a substance inhibiting tyrosinase activity such as kojic acid, kojic acid derivates etc., have an effect to inhibit the melanin generation without being accompanied by side effects such as leucoderma as observed with the hydroquinone etc. and are excellent topical agents for treating the pigmentation etc.; however, since they are those inhibiting an enzymatic effect to generate the melanin, they cannot yet be essentially satisfactory for the inhibition of the melanin generation.

The present invention has an object to obtain agents for satisfactorily inhibiting the melanin generation in the topical agents employed for the topical therapy for the pigmentation etc.

The present inventors have been intensively studying in order to achieve the above described object, and, as a result, have confirmed a surprising fact that when vitamin E and/or a vitamin E derivative are/is dissolved or suspended in an aqueous phase part to prepare a topical preparation, the aqueous solution of vitamin E or the vitamin E derivative is extremely excellent in the effect to inhibit the melanin generation while not inhibiting the tyrosinase activity, by a test using B16 cells derived from mouse melanoma.

It has been also discovered that this effect utterly differs from the conventional tyrosinase activity inhibiting agents used as melanin dye decolorizing agents such as kojic acid etc. and there is imparted an effect of inhibiting the melanin generation by the transfer interference in tyrosinase maturation processes, base on the histological and biochemical observation of the B16 cultured cells, thereby the present invention has been accomplished.

2

0 241 573

## Summary of the Invention

The present invention is a topical agent for inhibiting the melanin generation without inhibiting any tyrosinase activity, which has enabled easy skin absorption and cellular access to the target site, and which is characterized by incorporating vitamin E and/or a vitamin E derivative in a dissolved or suspended state in an aqueous phase part of the topical agent.

The topical agents of the present invention incorporate vitamin E or a vitamin E derivative in an aqueous phase in a preparation base in an aqueous solution or suspension state, and therefore, when the present topical agents are applied to the skin lesion, the vitamin E or vitamin E derivative penetrates through the epidermis, is absorbed by melanocytes in the epidermic basal strutum and remarkably inhibit the melanin generation by interfering the transfer of tyrosinase to premelanosome within the melanocytes. The topical agents for inhibiting the melanin generation which have enabled easy absorption through the skin and cell access to the target site without inhibiting any tyrosinase activity have been discovered by the present invention for the first time. Thus, the topical agents of the present invention are extremely effective in the prophylaxis and therapy of pigmentation such as chloasma etc.

The vitamin E and vitamin E derivatives used in the present invention include natural vitamin E oil, synthetic vitamin E such as $\alpha$-tocopherol, $\beta$-tocopherol, $\gamma$-tocopherol, $\delta$-tocopherol etc., vitamin E derivatives and salts thereof such as vitamin E acetate, vitamin E succinate, tocopherol nicotinate etc. The topical agents may be in forms such as emulsions, lotions, ointments etc., and may be used for the therapy or prophylaxis of the disease by coating such agents on the lesion.

In order to dissolved the above described vitamin E and vitamin E derivatives either singly or as a mixture of two or more thereof in an aqueous phase part of the aforementioned topical agent, they may be mixed with the aqueous phase part of the topical agent either by making an aqueous solution by a method which comprises solubilizing vitamin E and/or a vitamin E derivative in water by forming a liposome thereof together with a phospholipid, a method which comprises solubilizing vitamin E and/or a vitamin E derivative in water in the presence of a surfactant or a vitamin E solubilizer such as saponins, e.g., quillaja saponin, etc., or by making an organic solvent solution of vitamin E or a vitamin E derivative dissolved in an organic solvent. The above described vitamin E and vitamin E derivative may be used as the topical agents for the present invention even if not completely dissolved in the aqueous phase but partially in a suspended state as long as the vitamin E etc. are not separated in the phase, and also the effect thereof may satisfactorily be exerted. Further, it is also possible to incorporate them in the aqueous phase part of the topical agent in a suspended state. In this case, as a suspending agent, a surfactant, a phospholipid etc. may be used.

Where the aforementioned vitamin E and/or vitamin E derivative are/is to be solubilized in water and when a liposome of the vitamin E or vitamin E derivative is formed, a liposome containing vitamin E may be prepared by using a phospholipid such as natural lecithin such as egg yolk lecithin, soybean lecithin etc. and synthetic lecithin such as distearoyl lecithin etc. As the process for the production thereof, it may be produced by such conventional processes as a process which comprises dissolving a phospholipid with a vitamin E in a solvent such as chloroform, benzene etc., distilling off the solvent under reduced pressure using a rotary evaporator, adding an aqueous buffer to the formed thin film and vigorously shaking to swell to produce a multi-layered liposome, and further irradiating with ultrasonic waves to produce a mono-layered liposome, a process which comprises dissolving a phospholipid and a vitamin E in an organic solvent, and adding to a stirred aqueous buffer to produce a liposome, a process which comprises dissolving a phospholipid and a vitamin E in a solvent such as chloroform, benzene etc., adding thereto an aqueous buffer, emulisfying into a water-in-oil type emulsion by ultrasonic waves, distilling off the solvent by a rotary evaporator, and phase inverting into an oil-in-water type emulsion by voltex to produce a liposome, etc.

Where the vitamin E or vitamin E derivative is to be solubilized in water using a surfactant, there are used for example nonionic surfactants such as polyethylene glycol fatty acid esters, polyoxyethylene sorbitan fatty acid esters, polyoxyethylene glycerin fatty acid esters, polyglycerin fatty acid esters, polyoxyethylene castor oil, polyoxyethylene phytosterol, polyoxyethylene lanoline derivatives, polyoxyethylene alkyl ethers, polyoxyethylene polyoxypropylene alkyl ethers etc., and anionic surfactants such as alkyl ether phosphoric acids etc., and those having a HLB of 15 or higher are suitable. Further, the degree of solubilization is further enhanced by adding ethanol, propylene glycol etc. to the above described surfactants.

Further, where the vitamin E or vitamin E derivative is to be suspended, it is conducted by adding the above described phospholipids, surfactants etc. as a suspending agent.

3

Where the aqueous solution or aqueous suspension of the vitamin E or vitamin E derivative prepared as above is to be formulatd into a topical agent, it is formulated by a method conventionally employed for producing the above described preparations by using a base conventionally employed for emulsions, lotions, ointments etc.

In these cases, the aqueous solution or suspension of the vitamin E or vitamin E derivative is incorporated into the aqueous phase part of said preparations. The amount of the vitamin E or vitamin E derivative incorporated in the formulation is preferable 0.1-3.0% (w/w) based on the total amount of the topical agent.

Description of the Drawing

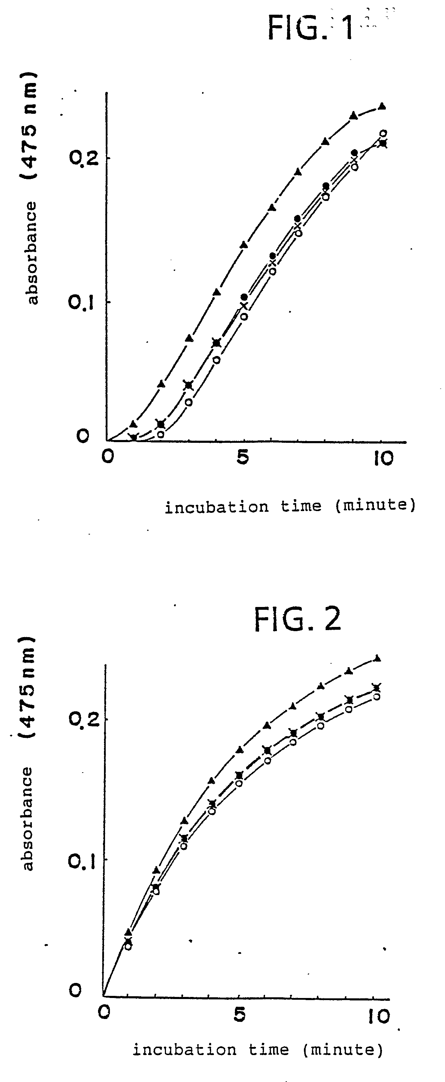

Fig. 1 is a figure showing the test results of the tyrosinase activity inhibition by vitamin E solutions on mashroom tyrosinase. In the figure, -▲-stands for the absorbance at 475 nm of an aqueous solution of a concentration of vitamin E of 0.1 μl/ml, -●-for an aqueous solution of concentration of vitamin E of 0.05 μl/ml, -x-for an aqueous solution of a concentration of vitamin E of 0.03 μl/ml and -o-for an aqueous solution containing no vitamin E (control).

Fig. 2 is a figure showing the test results of the tyrosinase activity inhibition by vitamin E solutions on tyrosinase from a B16 cell homogenate. In the figure, -▲-, -●-,-x-and -o-have the same significance as in Fig. 1.

Description of the Preferred Embodiments

Test examples showing the inhibition of the melanin generation by the topical agents for inhibiting the melanin generation of the present invention are shown below.

I. Test on B16 Cultured Cells Derived from Mouse Melanoma (Testing Method)

Aqueous solution of vitamin E was added 30 μg/ml, 20 μg/ml, 10 μg/ml and 2 μg/ml respectively in final concentration to the culture medium for B16 cells derived from the mouse melanoma. After culture period for 5 days at 37°C, the degree of melanin generation (degree of blackening) was observed by the naked eyes.

(Test Results)

| Concentration of Vitamin E | 0 | 2 μg/ml | 10 μg/ml | 20 μg/ml | 30 μg/ml |
|---|---|---|---|---|---|
| Degree of Whitening | − | ± | + | ++ | +++ |

Degree of Whitening-: Not whitened at all
+: Slightly whitened
+: Considerably whitened
+ +: Whitening clearly descernible
+ + +: Whitening proceeded to such degree that blackening cannot be observed

From these results, it can be seen that the B16 melanoma cells proceed the degree of whitening in proportion to the amount of vitamin E added.

II. Test in which the Ointment of the Present Invention was Applied to the Human Skin

In each of three hospitals, A, B and C, a test of the ointment application was conducted on 30 chloasma patients. The final judgment was determined by observing by the naked eyes 3 months after the start of the application.

(Test Results)

| Name of Hospital | A | B | C | Total |
|---|---|---|---|---|
| Non-effective | 6 | 6 | 14 | 16 |
| Slightly effective | 7 | 4 | 8 | 19 |
| Effective | 12 | 15 | 14 | 41 |
| Remarkably effective | 5 | 5 | 4 | 14 |
| Total | 30 | 30 | 30 | 90 |

(Notes)

Non-effective: That showing no whitening

Slightly effective: That showing slight whitening

Effective: That clearly showing whitening

Remarkably effective: That showing complete or almost cure

From the above results, of 90 in total in the three hospitals, there were 16 non-effective cases, 19 slightly effective cases, 4 effective cases and 14 remarkabley effective cases. The sum of those slightly effective to remarkably effective was 74, and thus the degree of effectiveness was 82.2%.

III. Test on the Tyrosinase Activity Inhibition by Vitamin E-containing Solutions

a. Test on the Mashroom-derived Tyrosinase Activity Inhibition

(Testing Method)

Samples were prepared by adding 1.0 ml of aqueous solutions containing vitamin E at concentrations of 0.1 µl/ml, 0.05 µl/ml and 0.03 µl/ml respectively to 1.0 ml of a tyrosine solution (0.3 mg/ml), 0.9 ml of a 0.1 M phosphate buffer (pH 6.8) and 0.1 ml of mashroom tyrosinase (400 U/ml), also a solution containing no vitamin E was prepared as a control, and the increase in absorbance at 475 nm at 37°C for 0-15 minutes was measured.

(Test Results)

The results of the present test are shown in Fig. 1.

As is clear from these test results, the effect to inhibit the mashroom tyrosinase by the vitamin E-containing solutions was not observed.

b. Test on the B16 cell-derived tyrosinase Activity Inhibition

5

(Testing Method)

Samples were prepared respectively by adding 1.0 ml of aqueous solutions containing vitamin E at concentrations of 0.1 $\mu$l/ml, 0.05 $\mu$l/ml and 0.03 $\mu$l/ml prepared in the production examples described hereinbelow to 0.1 ml of a 11000 G supernatant of a B16 cell homogenate, 1.0 ml of a 10 mM dopa and 0.9 ml of a 0.1 M phosphate buffer (pH 6.8), also a solution containing no vitamin E was prepared as a control, and the increase in absorbance at 475 nm at 37°C for 0.15 minutes was measured.

(Test Results)

The results of the present test are shown in Fig. 2.
As is clear from these results, the effect to inhibit the B16 cell tyrosinase by the vitamin E-containing solutions was not observed.

(Discussion)

To discuss the results of the respective tests described above, the effect to inhibit the melanin generation by the topical agents of the present invention is extremely excellent. It is obvious from the results of the above test III showing not tyrosinase activity inhibition that its active ingredient, i.e., vitamin E or a derivative thereof, does not act based on the effect to inhibit the melanin generation by the effect and function to inhibit the tyrosinase activity as exhibited by the conventional substances having a melanin inhibiting effect. It is suggested that the inhibition of the melanin generation by the present invention is an effect to inhibit the melanin generation by the inhibition of the tyrosinase transfer to premelanosome in the melanocytes present in the basal layer in the epidermis.
This is by an effect and function completely different from the effect by the conventional tyrosinase activity inhibition.

[Examples]

The production examples of vitamin E-and vitamin E derivative-containing aqueous solutions which are active ingredients for the topical agents of the present invention and the examples of the topical agents of the present invention are given below.

Production Example 1

Egg yolk lecithin and natural vitamin E are dissolved in ethanol and heated to 45°C.
Thereafter, the above described solution containing the egg yolk lecithin and natural vitamin E is poured with stirring into purified water heated to about 40°C and, after cooling to room temperature, filtered using a membrane filter to obtain a vitamin E liposome solution.

Production Example 2

One gram of dl-$\alpha$-tocopherol acetate is added to 5 g of polyoxyethlene hydrogenated castor oil (100 E.O.), heated to about 80°C, then added to 96 g of purified water heated to about 80°C, stirred and cooled to obtain a clear solution.

Production Example 3

Two grams of dl-$\alpha$-tocopherol is added to 3 g of polyoxyethylene sorbitan monooleate (20 E.O), heated to about 80°C, added to 95 g purified water heated to about 80°C, stirred and cooled to obtain an emulsion-like solution.

Example 1 (Ointment)

2.00 g of polyethylene glycol monostearate ( 40 E. 0.), 5.00 g of self emulsifing glycerin monostearate, 5. 00 g of stearic acid, 1.00 g of benenyl alcohol, 10.00 g of liquid paraffin, 10.00 g of glyceryl trioctanoate and 0.20 g of paraoxybenzoic acid methyl ester are heated and dissolved. A heated solution of 5.00 g of 1, 3-butylene glycol and 42.0 g of purified water is added thereto, emulsified by stirring, and cooled. To the thus obtained emulsion is added 20.0 g of the solution produced in Production Example 2 and containing 0.2 g of the dl-α-tocopherol acetate and a very small amount of a perfume, mixed by stirring, cooled, filled into containers and tested to provide products.

Example 2 (Emulsion)

1.00 g of polyoxyethylene sorbitan monostearate (20 E.O.), 0.5 g of polyoxyethylene sorbit tetraoleate (60 E.O.), 1.00 g of lipophilic glycerin monostearate, 0.50 g of stearic acid, 0.50 g of behenyl alcohol, 4.00 g of avocado oil, 4.00 g of glyceryl trioctanoate and 0.20 g of paraoxybenzoic acid methyl ester are heated and dissolved. A heated solution comprising 75.00 g of a suspension containing 5.00 g of 1, 3-butylene glycol, 0. 14 g of xanthane gum and 1.5 g of the dl-α-tocopherol produced in Production Example 3 and 7.0 g of purified water is added thereto, emulsified by stirring and cooled. To this solution is added a very small amount of a perfume and mixed by stirring. The thus obtained solution is cooled, filled into containers and tested to provide products.

Example 3 (Lotion)

The natural vitamin E liposome produced in Production Example 1 using 1.0 g of natural vitamin E, 1.0 g of egg yolk lecithin and 8.0 g of ethanol together with 0.10 g of paraoxybenzoic acid methyl ester, 0.01 g of hyaluronic acid, a very small amount of a perfume and a balance of purified water to give a total amount of 100 g are mixed by stirring, filled into containers and tested to provide products.

The topical agents for inhibiting the melanin generation obtained in the present invention are appropriately used according to the condition of the disease, and, in general, their effect may be sufficiently exerted by applying to the lesion after cleansing three times a day.

Vitamin E which is the active ingredient of the present invention is reported to exhibit no acute toxicity according to WHO.

**Claims**

A topical agent for inhibiting the melanin generation which enables easy skin absorption and cellular access to the target site without inhibiting any tyrosinase activity comprising incorporating vitamin E and/or a vitamin E derivative in a dissolved or suspended state in an aqueous phase part of the topical agent, thereby enabling easy skin absorption and cellular access to the targe site.

FIG. 1

0 241 573

absorbance (475 nm)

incubation time (minute)

FIG. 2

absorbance (475 nm)

incubation time (minute)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 95, no. 9, November 1981, page 365, abstract no. 156366w, Columbus, Ohio, US; & JP-A-81 75 421 (KANEBO COSMETICS INC.) 22-06-1981 | 1 | A 61 K    7/42<br>A 61 K    7/48 |
| X | PATENTS ABSTRACTS OF JAPAN, vol. 6, no. 15 (C-89)[893], 28th January 1982; & JP-A-56 139 409 (KANEBOU KESHIYOUHIN K.K.) 30-10-1981 | 1 | |
| X | PATENTS ABSTRACTS OF JAPAN, vol. 5, no. 200 (C-84)[872], 18th December 1981; & JP-A-56 120 612 (KANEBOU KESHIYOUHIN K.K.) 22-09-1981 | 1 | |
| | | | TECHNICAL FIELDS SEARCHED (Int. Cl.4) |
| X | EP-A-0 158 090  (ISMAIL ROSHDY) * Claims 1-4,8,10,19;  examples 1-137;  page 7, lines 9-20; page 9, line 16 - page 10, line 24; page 11, lines 4-9 * | 1 | A 61 K |
| X | SOAP/COSMETICS/CHEMICAL SPECIALTIES, vol. 48, no. 8, August 1972, pages 40-42; H. GOLDSCHMIEDT: "Vitamin E in cosmetics" . * Pages 40-42 * | 1 | |

The present search report has been drawn up for all claims

| Place of search | Date of completion of the search | Examiner |
|---|---|---|
| THE HAGUE | 18-11-1986 | FISCHER J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82